(19) Europäisches Patentamt
European Patent Office
Office européen des brevets

(11) Veröffentlichungsnummer : **0 157 270**
**B1**

(12) # EUROPÄISCHE PATENTSCHRIFT

(45) Veröffentlichungstag der Patentschrift : **15.06.88**

(51) Int. Cl.⁴ : **C 07 D319/20**, C 07 D319/22

(21) Anmeldenummer : **85103102.1**

(22) Anmeldetag : **18.03.85**

(54) **Verfahren zur Herstellung von tetrachlorierten Benzo-1,4-dioxenen.**

(30) Priorität : **31.03.84 DE 3412079**

(43) Veröffentlichungstag der Anmeldung : **09.10.85 Patentblatt 85/41**

(45) Bekanntmachung des Hinweises auf die Patenterteilung : **15.06.88 Patentblatt 88/24**

(84) Benannte Vertragsstaaten : **CH DE FR GB IT LI**

(56) Entgegenhaltungen : **US-A- 3 749 677**

(73) Patentinhaber : **BAYER AG**
**Konzernverwaltung RP Patentabteilung**
**D-5090 Leverkusen 1 Bayerwerk (DE)**

(72) Erfinder : **Marhold, Albrecht, Dr.**
**Carl-Duisberg-Strasse 329**
**D-5090 Leverkusen 1 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Tetrachlorierte Benzo-1,4-dioxene mit mindestens einer elektronegativen Gruppe am Benzolkern sind aus US-A-3 749 677 bekannt. Gemäss der älteren, aber nicht vorveröffentlichten EP-A-0 137 175 erhält man tetrachlorierte Benzo-1,4-dioxene durch Umsetzen entsprechender 2,3-Dioxobenzo-1,4-dioxene mit Phosphorpentachlorid.

Es wurde nun ein Verfahren zur Herstellung von tetrachlorierten Benzo-1,4-dioxenen der Formel

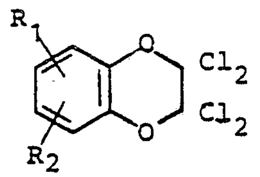

(I)

in der

$R_1$ und $R_2$ unabhängig voneinander für Wasserstoff, Fluor, Chlor, Brom, COCl, $CO_2CH_3$, Cyanid, Alkyl, Nitro, $SO_2Cl$, $SO_2F$, $OCF_3$, $SCF_3$, $CF_3$, $CCl_3$, $CBr_3$, Phenyl, substituiertes Phenyl, O-Alkyl, O-Aryl, S-Alkyl oder S-Aryl, oder in der

$R_1$ und $R_2$ gemeinsam für

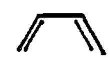

stehen, gefunden, das dadurch gekennzeichnet ist, daß man zunächst Brenzkatechin oder ein Brenzkatechinderivat der Formel

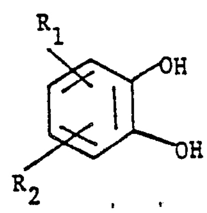

(II)

in der $R_1$ und $R_2$ die oben angegebene Bedeutung haben, Tetrachlorethylencarbonat und eine katalytische Menge eines tertiären Amins in beliebiger Reihenfolge bei 50 bis 100 °C in Gegenwart eines inerten Lösungsmittel zusammenbringt und nach Beendigung der stattfindenden Reaktion dem Reaktionsgemisch pro Mol eingesetzte Verbindung der Formel (II) mindestens 2 Mol Phosphorpentachlorid zufügt und dann das Reaktionsgemisch noch für einige Zeit bei erhöhter Temperatur beläßt.

Soweit in den Formeln (I) und (II) $R_1$ und/oder $R_2$ für substituiertes Phenyl stehen, kann es sich dabei beispielsweise um Phenylreste mit 6 bis 10 C-Atomen handeln, die durch Nitro, Fluor, Chlor, Brom, Cyanid, $C_1$- bis $C_4$-Alkyl, $SO_2Cl$, $SO_2F$, $OCF_3$, $SCF_3$, $CF_3$, $CCl_3$, $CBr_3$, O—$C_1$- bis $C_4$-Alkyl oder S—$C_1$- bis $C_4$-Alkyl substituiert sein können.

Soweit in den Formeln (I) und (II) $R_1$ und/oder $R_2$ für Alkyl, O-Alkyl und/oder S-Alkyl stehen, kann der jeweilige Alkylrest beispielsweise 1 bis 4 C-Atome enthalten.

Soweit in den Formeln (I) und (II) $R_1$ und/oder $R_2$ für O-Aryl und/oder S-Aryl stehen, kann der jeweilige Arylrest beispielsweise 6 bis 10 C-Atome enthalten.

Vorzugsweise stehen in den Formeln (I) und (II) $R_1$ und $R_2$ unabhängig voneinander für Wasserstoff, Fluor, Chlor, Methyl, Nitro oder Phenyl, oder es stehen $R_1$ und $R_2$ gemeinsam für

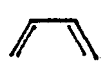

Besonders bevorzugt stehen in den Formeln (I) und (II) $R_1$ für Wasserstoff, $R_2$ für Wasserstoff, Nitro, Methyl oder Phenyl, oder es stehen $R_1$ und $R_2$ gemeinsam für

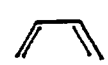

Die in das erfindungsgemäße Verfahren einzusetzenden Verbindungen der Formel (II) sind entweder im Handel erhältlich oder auf einfache Weise zugänglich, beispielsweise entsprechend Houben-Weyl,

Methoden der organischen Chemie, Band VI/1c, S. 327 ff. (1976) oder dazu analogen Verfahren.

Das in das erfindungsgemäße Verfahren einzusetzende Tetrachlorethylencarbonat ist beispielsweise durch Chlorierung von Ethylencarbonat gemäß der US-PS 2 815 287 erhältlich.

Bezogen auf die eingesetzte Verbindung der Formel (II) wird Tetrachlorethylencarbonat vorzugsweise in einem molaren Überschuß eingesetzt, beispielsweise in einer Menge von 1,01 bis 2 Mol, bezogen auf 1 Mol der Verbindung der Formel (II). Besonders bevorzugt ist der Einsatz von 1,1 bis 1,5 Mol Tetrachlorethylencarbonat, bezogen auf 1 Mol der Verbindung der Formel (II).

Als tertiäres Amin kommt beispielsweise Trimethylamin, Triethylamin, Dimethyl-phenylamin, Pyridin, Lutidin, 4-Dimethylamino-pyridin, Collidin, Chinolin, 1,8-Diazabicyclo-[5,4,0]-undec-7-en (= DBU) und 1,5-Diazabicyclo-[4,3,0]-non-5-en (= DBN) in Frage. Bevorzugt sind Pyridin und Pyridin-ähnliche Amine. Das tertiäre Amin kann beispielsweise in Mengen von 0,1 bis 5 g, bezogen auf 100 g Tetrachlorethylencarbonat, eingesetzt werden.

Als Lösungsmittel für das erfindungsgemäße Verfahren sind beispielsweise chlorierte Kohlenwasserstoffe geeignet, insbesondere solche mit einem Siedepunkt von über 60 °C, wie Chloroform, Tetrachlormethan, Chlorbenzol oder Dichlorbenzol.

Das Zusammenfügen der Verbindung der Formel (II), des Tetrachlorethylencarbonats, des tertiären Amins und des Lösungsmittels kann in beliebiger Reihenfolge durchgeführt werden. Beispielsweise kann man die Verbindung der Formel (II) vorlegen, dann das tertiäre Amin gelöst in einem Lösungsmittel und dann das Tetrachlorethylencarbonat zufügen. Man kann auch das tertiäre Amin zusammen mit dem Lösungsmittel vorlegen, dann langsam Tetrachlorethylencarbonat zugeben, die einsetzende Reaktion abklingen lassen und danach die Verbindung der Formel (II) zufügen. Man kann auch die Verbindung der Formel (II) zusammen mit dem Lösungsmittel vorlegen, Tetrachlorethylencarbonat zufügen und schließlich das tertiäre Amin.

Die Umsetzung der Verbindung der Formel (II) mit Tetrachlorethylencarbonat oder mit dem nach der Reaktion von Tetrachlorethylencarbonat mit dem tertiären Amin vorliegenden Gemisch wird bei 50 bis 100 °C durchgeführt. Vorzugsweise liegt diese Temperatur bei 65 bis 85 °C. Diese Reaktion ist beendet, wenn die Chlorwasserstoff-Entwicklung zum Stillstand kommt, was im allgemeinen nach etwa 2 bis 10 Stunden der Fall ist.

Es ist ein wesentliches Merkmal der vorliegenden Erfindung, daß man nunmehr keine Isolierung der Reaktionsprodukte vornimmt, sondern direkt in das vorliegende Reaktionsgemisch mindestens 2 Mole Phosphorpentachlorid pro Mol eingesetzter Verbindung der Formel (II) zufügt. Größere Mengen Phosphorpentachlorid stören im allgemeinen nicht. Aus wirtschaftlichen Erwägungen setzt man vorzugsweise 2 bis 3 Mol Phosphorpentachlorid pro Mol eingesetzter Verbindung der Formel (II) ein.

Nach Zugabe des Phosphorpentachlorids hält man das Reaktionsgemisch noch 0,5 bis 10 Stunden bei 60 bis 150 °C. Bevorzugt sind hier Reaktionszeiten von 2 bis 6 Stunden und Temperaturen von 65 bis 120 °C.

Die Aufarbeitung des dann vorliegenden Gemisches kann beispielsweise so erfolgen, daß man zunächst das gebildete Phosphoroxichlorid und das Lösungsmittel abdestilliert, wobei gegebenenfalls vorhandenes überschüssiges Phosphorpentachlorid sublimiert, und anschließend den verbleibenden Rückstand einer Fraktionierten Destillation im Vakuum unterwirft. Eine weitere Reinigung der erhaltenen Verbindungen der Formel (I) kann gegebenenfalls durch Umkristallisation vorgenommen werden. Eine solche Umkristallisation kann beispielsweise aus Kohlenwasserstoffen, wie Cyclohexan, Toluol oder Xylol, oder aus Alkoholen, wie Methanol oder Ethanol erfolgen.

Mit dem erfindungsgemäßen Verfahren sind die Verbindungen der Formel (I) auf einfache Weise und in guten Ausbeuten und Selektivitäten zugänglich.

Die Verbindungen der Formel (I) sind wertvolle Produkte, aus denen sich beispielsweise durch eine an sich bekannte Fluorierung mit Fluorwasserstoff und gegebenenfalls anschließende Nitrierung und Reduktion Aniline herstellen lassen, aus denen durch Umsetzung mit Acylisocyanaten Verbindungen zugänglich sind, die gemäß den DE-OS'en 30 23 328 und 32 23 505 eine überlegene insektizide Wirkung zeigen.

Die folgenden Beispiele erläutern das erfindungsgemäße Verfahren ohne es in irgendeiner Weise zu beschränken.

## Beispiele

### Beispiel 1

Es wurden 400 ml trockenes Chlorbenzol und 2 ml Pyridin vorgelegt und auf 65 bis 70 °C erwärmt. Danach wurden 320 g Tetrachlorethylencarbonat zugetropft. Es setzte sofort eine Gasentwicklung ein. Nach 3 Stunden war die Gasentwicklung bei 75 bis 80 °C beendet. Danach wurden 110 g Brenzkatechin über eine Substanzbirne eingetragen, wobei sofort eine heftige Chlorwasserstoffentwicklung einsetzte. Die Temperatur wurde bei 80 °C gehalten. Nach Ende der Zugabe wurde noch 3 Stunden bei 80 °C nachgerührt. Dann hatte die Gasentwicklung aufgehört, und nach gaschromatographischer Analyse war das gesamte Brenzkatechin umgesetzt. Nunmehr wurden 480 g Phosphorpentachlorid zugegeben und das Gemisch 4 Stunden auf Rückflußtemperatur erhitzt. Danach wurde Phosphoroxychlorid und

Chlorbenzol abdestilliert, wobei überschüssiges Phosphorpentachlorid sublimierte. Die Destillation des verbliebenen Rückstandes lieferte 23 g eines Vorlaufs mit einem Siedepunkt von 95 bis 118 °C bei 8 mbar, 220 g Tetrachlorbenzodioxen mit einem Siedepunkt von 118-130 °C bei 8 mbar und 19 g eines nicht näher untersuchten Rückstandes.

## Beispiel 2

In 1 200 ml Chlorbenzol wurden 440 g Brenzkatechin und 3 ml Pyridin vorgelegt. Anschließend wurde bei 75 °C 1 000 g Tetrachlorethylencarbonat zugetropft. Während der Reaktion bei 75 bis 80 °C wurden jede Stunde 0,5 ml Pyridin zudosiert. Nach Beendigung der Chlorwasserstoff-Entwicklung wurden 1 900 g Phosphorpentachlorid zugegeben und das Gemisch für 5 Stunden auf Rückflußtemperatur erhitzt. Danach wurde fraktioniert destilliert. Es wurden 585 g Tetrachlorbenzodioxen erhalten.

## Beispiele 3 bis 6

Es wurde verfahren wie in Beispiel 2, jedoch wurden anstelle von Brenzkatechin Brenzkatechinderivate eingesetzt. Die Ergebnisse sind aus Tabelle 1 ersichtlich.

Tabelle 1

| Beispiel Nr. | Reaktionsprodukt 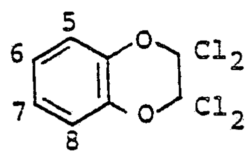 | Ausbeute (d.Th.) | Siede- oder Schmelzpunkt (°C) |
|---|---|---|---|
| 3 | 6-Nitro | 74 | 81-82 (Schmp.) |
| 4 | 5-Methyl | 70 | 158-162/ 20 mbar (Sdp.) |
| 5 | 6-Phenyl | 78 | 115-117 (Schmp.) |
| 6 | 6,7- 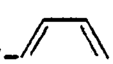 | 83 | 175-178 (Schmp.) |

## Patentansprüche

1. Verfahren zur Herstellung von tetrachlorierten Benzo-1,4-dioxenen der Formel

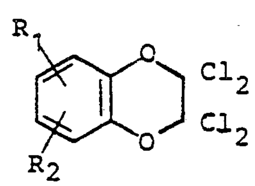

in der

$R_1$ und $R_2$ unabhängig voneinander für Wasserstoff, Fluor, Chlor, Brom, COCl, $CO_2CH_3$, Cyanid, Alkyl, Nitro, $SO_2Cl$, $SO_2F$, $OCF_3$, $SCF_3$, $CF_3$, $CCl_3$, $CBr_3$, Phenyl, substituiertes Phenyl, O-Alkyl, O-Aryl, S-Alkyl oder S-Aryl, oder in der $R_1$ und $R_2$ gemeinsam für

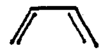

stehen, dadurch gekennzeichnet, daß man zunächst Brenzkatechin oder ein Brenzkatechinderivat der Formel

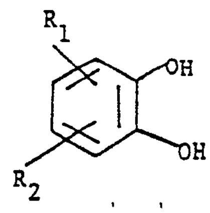

in der $R_1$ und $R_2$ die oben angegebene Bedeutung haben, Tetrachlorethylencarbonat und eine katalytische Menge eines tertiären Amins in beliebiger Reihenfolge bei 50 bis 100 °C in Gegenwart eines inerten Lösungsmittel zusammenbringt und nach Beendigung der stattfindenden Reaktion dem Reaktionsgemisch pro Mol eingesetztes Brenzkatechin oder Brenzkatechinderivat mindestens 2 Mol Phosphorpentachlorid zufügt und dann das Reaktionsgemisch noch für einige Zeit bei erhöhter Temperatur beläßt.

2. Verfahren nach Anspruch 1, dadurch gekennzeichnet, daß man Brenzkatechin oder ein Brenzkatechinderivat der angegebenen Formel einsetzt, bei der $R_1$ und $R_2$ unabhängig voneinander für Wasserstoff, Fluor, Chlor, Methyl, Nitro oder Phenyl, oder bei der $R_1$ und $R_2$ gemeinsam für

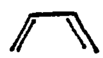

stehen.

3. Verfahren nach Ansprüchen 1 und 2, dadurch gekennzeichnet, daß man 1,01 bis 2 Mol Tetrachlorethylencarbonat, bezogen auf 1 Mol Brenzkatechin oder Brenzkatechinderivat, einsetzt.

4. Verfahren nach Ansprüchen 1 bis 3, dadurch gekennzeichnet, daß man als tertiäres Amin Trimethylamin, Triethylamin, Dimethyl-phenylamin, Pyridin, Lutidin, 4-Dimethylamino-pyridin, Collidin, Chinolin, 1,8-Diazabicyclo-[5,4,0]-undec-7-en oder 1,5-Diazabicyclo-[4,3,0]-non-5-en, einsetzt.

5. Verfahren nach Ansprüchen 1 bis 4, dadurch gekennzeichnet, daß man das tertiäre Amin in Mengen von 0,1 bis 5 g, bezogen auf 100 g Tetrachlorethylencarbonat, einsetzt.

6. Verfahren nach Ansprüchen 1 bis 5, dadurch gekennzeichnet, daß man als Lösungsmittel chlorierte Kohlenwasserstoffe mit einem Siedepunkt über 60 °C einsetzt.

7. Verfahren nach Ansprüchen 1 bis 6, dadurch gekennzeichnet, daß man 2 bis 3 Mol Phosphorpentachlorid pro Mol Brenzkatechin oder Brenzkatechinderivat einsetzt.

8. Verfahren nach Ansprüchen 1 bis 7, dadurch gekennzeichnet, daß man das Reaktionsgemisch nach der Zugabe von Phosphorpentachlorid noch 0,5 bis 10 Stunden bei 60 bis 150 °C hält.

9. Verfahren nach Ansprüchen 1 bis 8, dadurch gekennzeichnet, daß man das Reaktionsgemisch nach der Zugabe von Phosphorpentachlorid noch 2 bis 6 Stunden bei 65 bis 120 °C hält.

10. Verfahren nach Ansprüchen 1 bis 9, dadurch gekennzeichnet, daß man nach Beendigung der Reaktion mit Phosphorpentachlorid das gebildete Phosphoroxichlorid und das Lösungsmittel abdestilliert und anschließend den verbleibenden Rückstand einer fraktionierten Destillation im Vakuum unterwirft.

**Claims**

1. Process for the preparation of tetrachlorinated benzo-1,4-dioxenes of the formula

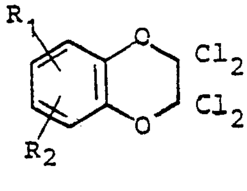

in which

$R_1$ and $R_2$ independently of one another represent hydrogen, fluorine, chlorine, bromine, COCl, $CO_2CH_3$, cyanide, alkyl, nitro, $SO_2Cl$, $SO_2F$, $OCF_3$, $SCF_3$, $CF_3$, $CCl_3$, $CBr_3$, phenyl, substituted phenyl, O-alkyl, O-aryl, S-alkyl or S-aryl, or

in which

$R_1$ and $R_2$ together represent

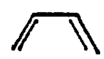

characterised in that pyrocatechol or a pyrocatechol derivative of the formula

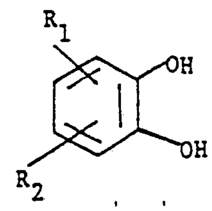

in which $R_1$ and $R_2$ have the abovementioned meaning, is first brought together with tetrachloroethylene carbonate and a catalytic amount of a tertiary amine in any desired sequence at 50 to 100 °C in the presence of an inert solvent, and, when the reaction which takes places has ended, at least 2 moles of phosphorus pentachloride are added to the reaction mixture per mole of pyrocatechol or pyrocatechol derivative employed, and the reaction mixture is then left at an elevated temperature for a further period.

2. Process according to Claim 1, characterised in that pyrocatechol or a pyrocatechol derivative of the given formula in which $R_1$ and $R_2$ independently of one another represent hydrogen, fluorine, chlorine, methyl, nitro or phenyl, or in which $R_1$ and $R_2$ together represent

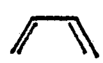

is employed.

3. Process according to Claims 1 and 2, characterised in that 1.01 to 2 moles of tetrachloroethylene carbonate are employed per mole of pyrocatechol or pyrocatechol derivative.

4. Process according to Claims 1 to 3, characterised in that trimethylamine, triethylamine, dimethylphenylamine, pyridine, lutidine, 4-dimethylaminopyridine, collidine, quinoline, 1,8-diazabicyclo-[5,4,0]-undec-7-ene or 1,5-diazabicyclo-[4,3,0]-non-5-ene is employed as the tertiary amine.

5. Process according to Claims 1 to 4, characterised in that the tertiary amine is employed in amounts of 0.1 to 5 g per 100 g of tetrachloroethylene carbonate.

6. Process according to Claims 1 to 5, characterised in that chlorinated hydrocarbons with a boiling point above 60 °C are employed as the solvent.

7. Process according to Claims 1 to 6, characterised in that 2 to 3 moles of phosphorus pentachloride are employed per mole of pyrocatechol or pyrocatechol derivative.

8. Process according to Claims 1 to 7, characterised in that the reaction mixture is kept at 60 to 150 °C for a further 0.5 to 10 hours after the addition of phosphorus pentachloride.

9. Process according to Claims 1 to 8, characterised in that the reaction mixture is kept at 65 to 120 °C for a further 2 to 6 hours after the addition of phosphorus pentachloride.

10. Process according to Claims 1 to 9, characterised in that, when the reaction with phosphorus pentachloride has ended, the phosphorus oxychloride formed and the solvent are distilled off and the residue which remains in then subjected to fractional distillation in vacuo.

## Revendications

1. Procédé de préparation de benzo-1,4-dioxènes tétrachlorés de formule

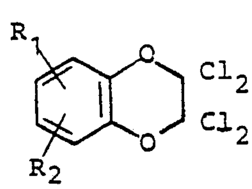

dans laquelle

$R_1$ et $R_2$ représentent chacun, indépendamment l'un de l'autre, l'hydrogène, le fluor, le chlore, le brome, un groupe COCl, $CO_2CH_3$, cyanure, alkyle, nitro, $SO_2Cl$, $SO_2F$, $OCF_3$, $SCF_3$, $CF_3$, $CCl_3$, $CBr_3$, phényle, phényle substitué, O-alkyle, O-aryle, S-alkyle ou S-aryle, ou dans laquelle

$R_1$ et $R_2$ représentent ensemble

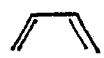

caractérisé en ce que l'on met d'abord en contact dans un ordre quelconque, à 50-100 °C, en présence d'un solvent inerte, le pyrocatéchol ou un dérivé du pyrocatéchol de formule

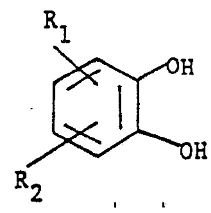

dans laquelle $R_1$ et $R_2$ ont les significations indiquées ci-dessus, du carbonate de tétrachloréthylène et une quantité catalytique d'une amine tertiaire et, lorsque la réaction est terminée, on ajoute au mélange de réaction, pour 1 mole de pyrocatéchol ou de dérivé de pyrocatéchol mis en œuvre, au moins 2 moles de pentachlorure de phosphore puis on maintient le mélange de réaction pendant encore quelque temps à chaud.

2. Procédé selon la revendication 1, caractérisé en ce que l'on utilise le pyrocatéchol ou un dérivé du pyrocatéchol de formule ci-dessus dans laquelle $R_1$ et $R_2$ représentent chacun, indépendamment l'un de l'autre, l'hydrogène, le fluor, le chlore, un groupe méthyle, nitro ou phényle, ou bien dans laquelle $R_1$ et $R_2$ représentent ensemble

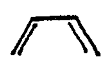

3. Procédé selon les revendications 1 et 2, caractérisé en ce que l'on utilise 1,01 à 2 moles de carbonate de tétrachloréthylène par mole de pyrocatéchol ou de dérivé de pyrocatéchol.

4. Procédé selon les revendications 1 à 3, caractérisé en ce que l'on utilise en tant qu'amine tertaire la triméthylamine, la triéthylamine, la diméthylphénylamine, la pyridine, la lutidine, la 4-diméthylaminopyridine, la collidine, la quinoléine, le 1,8-diazabicyclo-[5,4,0]-undéca-7-ène ou le 1,5-diazabicyclo-[4,3,0]-nona-5-ène.

5. Procédé selon les revendications 1 à 4, caractérisé en ce que l'on utilise l'amine tertiaire en quantité de 0,1 à 5 g pour 100 g de carbonate de tétrachloréthylène.

6. Procédé selon les revendications 1 à 5, caractérisé en ce que l'on utilise en tant que solvants des hydrocarbures chlorés bouillant à plus de 60 °C.

7. Procédé selon les revendications 1 à 6, caractérisé en ce que l'on utilise de 2 à 3 moles de pentachlorure de phosphore par mole de pyrocatéchol ou dérivé du pyrocatéchol.

8. Procédé selon les revendications 1 à 7, caractérisé en ce que, après l'addition du pentachlorure de phosphore, on maintient le mélange de réaction pendant encore 1/2 à 10 h à une température de 60 à 150 °C.

9. Procédé selon les revendications 1 à 8, caractérisé en ce que, après l'addition du pentachlorure de phosphore, on maintient le mélange de réaction pendant encore 2 à 6 h à une température de 65 à 120 °C.

10. Procédé selon les revendications 1 à 9, caractérisé en ce que, après la réaction avec le pentachlorure de phosphore, on distille l'oxychlorure de phosphore formé et le solvant puis on soumet le résidu à distillation fractionnée sous vide.